(19) Europäisches Patentamt
European Patent Office
Office européen des brevets

(11) Veröffentlichungsnummer: **0 291 805 B1**

# (12) EUROPÄISCHE PATENTSCHRIFT

(45) Veröffentlichungstag der Patentschrift: **04.12.91**

(51) Int. Cl.5: **C07C 47/56**, C07C 45/61

(21) Anmeldenummer: **88107402.5**

(22) Anmeldetag: **07.05.88**

## (54) Verfahren zur Herstellung von p-Hydroxymethylbenzaldehyd.

(30) Priorität: **19.05.87 DE 3716731**

(43) Veröffentlichungstag der Anmeldung:
**23.11.88 Patentblatt 88/47**

(45) Bekanntmachung des Hinweises auf die Patenterteilung:
**04.12.91 Patentblatt 91/49**

(84) Benannte Vertragsstaaten:
**BE DE ES FR GB IT NL**

(56) Entgegenhaltungen:
**US-A- 3 845 138**
**US-A- 3 987 041**

**CHEMICAL ABSTRACTS, Band 83, Nr. 7, 18. August 1975, Zusammenfassung Nr. 58958y; N.M. WEINSHENKER et al.: "Polymeric reagents. IV. Synthesis and utilisation of an insoluble polymeric organotin dihydride reagent"**

**CHEMICAL ABSTRACTS, Band 80, Nr. 19, 13. Mai 1974, Zusammenfassung Nr. 108110b; C.C. LEZNOFF et al.: "Use of polymer supports in organic synthesis. III. Selective chemical reactions on one aldehyde group of symmetrical dialdehydes"**

**CHEMICAL ABSTRACTS, Band 88, Nr. 22, 29. Mai 1978, Zusammenfassung Nr. 160551a; L. HORNER et al.: "Studies on the occurrence of hydrogen transfer, 47. Electroreduction of 1,4-benzenedicarbonitrile, 1,4-diacetylbenzene and terephthalaldehyde"**

**TETRAHEDRON LETTERS, Band 24, 1983, Nr. 40, Seiten 4287 - 4290, Pergamon Press, Oxford; C.F. NUTAITIS et al.: "Chemoselective reduction of aldehydes with tetra-n-butylammonium triacetoxyborohydride"**

(73) Patentinhaber: **BASF Aktiengesellschaft
Carl-Bosch-Strasse 38
W-6700 Ludwigshafen(DE)**

(72) Erfinder: **Wild, Jochen, Dr.
An der Marlach 7
W-6705 Deidesheim(DE)**
Erfinder: **Janssen, Bernd, Dr.
Leuschnerstrasse 18 a
W-6700 Ludwigshafen(DE)**
Erfinder: **Goetz, Norbert, Dr.
Schoefferstrasse 25
W-6520 Worms 1(DE)**

**EP 0 291 805 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von p-Hydroxymethylbenzaldehyd durch partielle katalytische Hydrierung von Terephthalaldehyd mit stöchiometrischen oder annähernd stöchiometrischen Mengen Wasserstoff in Gegenwart eines festen Hydrierkatalysators sowie in Gegenwart von Wasser und einer inerten, mit Wasser homogen mischbaren organischen Flüssigkeit.

Diese Reaktion

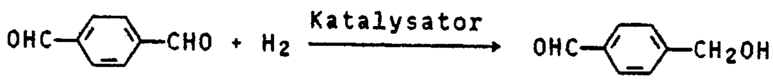


die in ihren Grundzügen aus der US-A-3 845 138 bekannt ist, hat den generellen Nachteil, daß sie zu Gemischen führt, die neben dem erwünschten p-Methylbenzylalkohol auch den zweifach hydrierten und den unveränderten Terephthalaldehyd sowie weitere Hydrierungsprodukte, z.B. p-Methylbenzylalkohol, enthalten, und zwar auch dann, wenn man mit stöchiometrischen Mengen Wasserstoff arbeitet.

Die Herstellung des p-Hydroxymethylbenzaldehyds ist daher stets mit Ausbeuteverlusten verbunden, ganz abgesehen davon, daß die Isolierung dieser empfindlichen Verbindung aus ihren Reaktionsgemischen einen erheblichen verfahrenstechnischen Aufwand erfordert.

Nach der Lehre der US-A cit. kann man die unerwünschte zweifache Hydrierung zurückdrängen, indem man den Terephthalaldehyd in Form einer organischen oder wäßrig-organischen Lösung einsetzt und die Hydrierung in Gegenwart einer löslichen Base vornimmt, deren Anwesenheit allerdings zusätzliche Aufarbeitungsschwierigkeiten verursacht.

Der Erfindung lag daher die Aufgabe zugrunde, die durch die Mitverwendung zusätzlicher Stoffe bedingten Nachteile zu beheben und das für organische Synthesen wichtige Zwischenprodukt p-Hydroxymethylbenzaldehyd einfacher und wirtschaftlicher zugänglich zu machen.

Demgemäß wurde ein Verfahren zur Herstellung von p-Hydroxymethylbenzaldehyd durch partielle katalytische Hydrierung von Terephthalaldehyd mit stöchiometrischen oder annähernd stöchiometrischen Mengen Wasserstoff in Gegenwart eines festen Hydrierkatalysators sowie in Gegenwart von Wasser und einer inerten, mit Wasser homogen mischbaren organischen Flüssigkeit gefunden, welches dadurch gekennzeichnet ist, daß man die Umsetzung in einem homogenen wäßrig-organischen Medium vornimmt, in welchem der Terephthalaldehyd größtenteils in suspendierter Form vorliegt, und daß man in praktischer Abwesenheit basischer Stoffe arbeitet.

Art und Menge des wäßrig-organischen Reaktionsmediums wählt man zweckmäßigerweise so, daß sich unter den Reaktionsbedingungen zu Beginn der Hydrierung hierin nicht mehr als 20, vorzugsweise nicht mehr als 10 Gew.% des Terephthalaldehyds lösen, und daß der entstehende p-Hydroxymethylbenzaldehyd vollständig in Lösung gehen kann, so daß man gegen Ende der Reaktion eine klare Lösung des p-Hydroxymethylbenzaldehyds erhält, in welcher sich als heterogener Bestandteil nur noch der Hydrierkatalysator befindet.

Wäßrig-organische Medien, welche diesen Bedingungen entsprechen, enthalten in der Regel mindestens 60 Vol.% Wasser und mindestens 5 Vol.% der wasserlöslichen organischen Flüssigkeit. Vorzugsweise setzen sich die Medien aus 65 bis 75 Vol.% Wasser und 25 bis 35 Vol.% der organischen Komponente zusammen.

Als wasserlösliche organische Flüssigkeiten, die sich unter den Reaktionsbedingungen selbstverständlich inert verhalten sollen, kommen vorzugsweise wasserlösliche Alkanole wie Methanol, Ethanol und Isopropanol, mehrwertige Alkohole wie Ethylenglykol und Diethylenglykol, cyclische Ether wie Tetrahydrofuran und Dioxan sowie daneben auch Dimethylformamid sowie Gemische dieser Flüssigkeiten in Betracht.

Im allgemeinen empfiehlt sich die Verwendung von billigen leichtsiedenden Flüssigkeiten wie Methanol und Ethanol, da diese vom Umsetzungsgemisch leicht abgetrennt werden können.

Die Menge des Hydriermediums wird zweckmäßigerweise so bemessen, daß einerseits möglichst wenig Terephthalaldehyd in Lösung geht, andererseits das Reaktionsgemisch aber noch leicht zu rühren ist. Im allgemeinen liegt diese Menge zwischen 2 und 20 l pro kg des Terephthalaldehyds.

Als Hydrierkatalysatoren eignen sich prinzipiell sämtliche Feststoffkontakte, die man für die heterogene Hydrierung von Carbonylfunktionen verwenden kann. Bevorzugt werden Metalle der VIII. Nebengruppe des Periodensystems wie Nickel, Cobalt und die Edelmetalle, insbesondere in Form von Trägerkatalysatoren.

Besonders gut geeignet sind Palladium/Aktivkohle-Katalysatoren mit etwa 2 bis 15 Gew.% Pd-Gehalt.

Die Menge der Katalysatoren ist nicht kritisch und richtet sich nach deren Art und Teilchengröße. Im allgemeinen liegt sie zwischen 1 und 30, vorzugsweise 5 und 20 g pro kg des Terephthalaldehyds.

Vorzugsweise nimmt man die Hydrierung bei 15 bis 30 °C vor. Unterhalb von etwa 5 °C verlangsamt sich die Reaktion zu sehr und oberhalb von 60 °C ist mit merklichen Nebenreaktionen zu rechnen.

Für den Wasserstoffpartialdruck kommt ein Bereich von etwa 0,1 bis 20 bar in Betracht, wobei sich für praktische Zwecke der Bereich von Normaldruck bis etwa 3 bar besonders empfiehlt.

Der Wasserstoff wird vorzugsweise in stöchiometrischer Menge zum Terephthalaldehyd eingesetzt, jedoch können auch in manchen Fällen annäherend stöchiometrische Mengen zweckmäßig sein, etwa wenn man zur schnelleren vollständigen Umsetzung des Terephthalaldehyds eine begrenzte Mehrbildung des p-Bis-p-hydromethylbenzols in Kauf nehmen will.

Die Maßgabe, man solle in praktischer Abwesenheit basischer Stoffe arbeiten, bedeutet, daß man dem Reaktionsgemisch solche Stoffe nicht gezielt oder nicht in Mengen zusetzt, in denen sie einen merklichen Einfluß auf die Hydrierung oder die Aufarbeitung haben.

Man kann die Umsetzung nach den üblichen Arbeitstechniken vornehmen, etwa indem man die katalysatorhaltige Suspension des Terepthalaldehyds vorlegt und den Wasserstoff nach Maßgabe seines Verbrauches bis etwa zur stöchiometrischen Menge unter Rühren in diese Suspension einleitet.

Die Aufarbeitung des Reaktionsgemisches, welche am Ende der Hydrierung als Lösung des Verfahrensproduktes im Reaktionsmedium vorliegt und als Feststoff nur noch den Katalysator enthält, gestaltet sich infolge der Abwesenheit sonstiger Stoffe besonders einfach, indem man lediglich den Katalysator abzufiltrieren und die verbleibende Lösung unter schonenden Bedingungen destillativ vom Wasser und vom Lösungsmittel zu befreien braucht.

Man erhält den p-Hydroxymethylbenzaldehyd in hoher Ausbeute bis zu 99 %, wobei man es zweckmäßigerweise durch leichte Überhydrierung so einrichtet, daß kein Terephthalaldehyd mehr vorliegt. Die geringen Mengen des als Nebenprodukt anfallenden Bishydromethylbenzols stören bei der weiteren Umsetzung des p-Hydroxymethylbenzaldehyds im allgemeinen nicht.

Das gute Gelingen des erfindungsgemäßen Verfahrens, welches von dem leicht zugänglichen Terephtaladehyds ausgeht, ist im Hinblick darauf besonders bemerkenswert, daß der p-Hydroxymethylbenzaldehyd praktisch nicht weiterhydriert wird, obwohl er im Gegensatz zum suspendierten Terephthalaldehyd in Lösung vorliegt und eine Reaktion mit gelösten Reaktionspartnern normalerweise schneller verläuft als mit festen Stoffen.

Der p-Hydroxymethylbenzaldehyd ist ein wertvolles Zwischenprodukt für organische Synthesen, u.a. zur Herstellung von Pflanzenschutzmitteln, Farbstoffen und Arzneimitteln.

Beispiel 1

20 g Terephthalaldehyd wurden in einer Mischung aus 200 ml Wasser und 80 ml Ethanol suspendiert. Diese Suspension wurde mit 0,5 g eines Palladium/Aktivkohlekatalysators (Pd-Gehalt 10 Gew.%) versetzt, wonach bei 25 °C solange Wasserstoff unter Atmosphärendruck eingeleitet wurde, bis gaschromatographisch kein Terephthalaldehyd mehr nachzuweisen war. Die Reaktionszeit betrug etwa 5 Stunden, und der Wasserstoffverbrauch lag etwas über der stöchiometrischen, zur partiellen Hydrierung erforderlichen Menge.

Nach Abtrennung des Katalysators wurde das Wasser-Ethanolgemisch bei 40 °C/10 mbar destillativ entfernt. Der Rückstand erstarrte nach einigen Stunden zu einer gelblichen kristallinen Masse, die aus 98,5 Gew.% p-Hydroxymethylbenzaldehyd und 1,5 Gew.% Bis-p-hydroxymethylbenzol bestand. Die Ausbeute an p-Hydroxymethylbenzaldehyd betrug 97,0 %.

Beispiel 2

Unter den Bedingungen von Beispiel 1, jedoch mit 70 ml Dioxan anstelle des Ethanols, fielen der p-Hydroxymethylbenzaldehyd bei vollständigem Umsatz des Terephthalaldehyds in einer Selektivität von 93 % und das Bis-p-hydroxymethylbenzol in einer Selektivität von 7 % an.

## Patentansprüche

1. Verfahren zur Herstellung von p-Hydroxymethylbenzaldehyd durch partielle katalytische Hydrierung von Terephthalaldehyd mit stöchiometrischen oder annähernd stöchiometrischen Mengen Wasserstoff in Gegenwart eines festen Hydrierkatalysators sowie in Gegenwart von Wasser und einer inerten, mit Wasser homogen mischbaren organischen Flüssigkeit, dadurch gekennzeichnet, daß man die Umsetzung in einem homogenen wäßrig-organischen Medium vornimmt, in welchem der Terephthalaldehyd größtenteils in suspendierter Form vorliegt, und daß man in praktischer Abwesenheit basischer Stoffe arbeitet.

2. Verfahren nach Anspruch 1, dadurch gekennzeichnet, daß das homogene wäßrig-organische Medium aus 60 bis 95 Vol.% Wasser und 5 bis 40 Vol.% Methanol, Ethanol, Isopropanol, Tetrahydrofuran und/oder Dioxan besteht.

## Claims

1. A process for the preparation of p-hydroxymethylbenzaldehyde by partial catalytic hydrogenation of terephthalaldehyde using a stoichiometric or approximately stoichiometric amount of hydrogen in the presence of a solid hydrogenation catalyst, water and an inert, homogeneously water-miscible organic liquid, which comprises carrying out the reaction in a homogeneous aqueous-organic medium in which the majority of the terephthalaldehyde is in suspended form, and working in the practical absence of basic substances.

2. A process as claimed in claim 1, wherein the homogeneous aqueous-organic medium comprises from 60 to 95% by volume of water and from 5 to 40% by volume of methanol, ethanol, isopropanol, tetrahydrofuran and/or dioxane.

## Revendications

1. Procédé de préparation du p-hydroxyméthylbenzaldéhyde par hydrogénation catalytique partielle de l'aldéhyde téréphtalique à l'aide d'hydrogène en quantité stoechiométrique ou voisine de la quantité stoechiométrique en présence d'un catalyseur d'hydrogénation solide et en présence d'eau et d'un liquide organique inerte entièrement miscible à l'eau, caractérisé en ce que l'on effectue la réaction dans un milieu hydro-organique homogène dans lequel l'aldéhyde téréphtalique est pour la plus grande part à l'état de suspension, et en ce que l'on opère pratiquement en l'absence de substances basiques.

2. Procédé selon la revendication 1, caractérisé en ce que le milieu hydro-organique homogène consiste en 60 à 95 % en volume d'eau et 5 à 40 % en volume de méthanol, d'éthanol, d'isopropanol, de tétrahydrofuranne et/ou de dioxanne.